# EUROPÄISCHE PATENTSCHRIFT

(11) **EP 2 670 490 B1**
(45) Veröffentlichungstag und Bekanntmachung des Hinweises auf die Patenterteilung: **27.06.2018**
(21) Anmeldenummer: 12702807.4
(22) Anmeldetag: 01.02.2012
(51) Int. Cl.: A61K 8/37, A61Q 15/00, A61K 8/86, A61K 8/891, A61K 8/92, A61K 8/04, A61K 8/22

(54) **DESODORIERENDER HAUTFILM**
SKIN DEODORIZING FILM
FILM CUTANÉ DÉODORANT

(30) Priorität: 02.02.2011 DE 102011003533
(43) Veröffentlichungstag der Anmeldung: 11.12.2013
(73) Patentinhaber: Beiersdorf AG, 20253 Hamburg (DE)
(72) Erfinder: BIEL, Stefan, 21077 Hamburg (DE); MEYER, Maren, 22459 Hamburg (DE)
(74) Vertreter: Hartmann, Jost
(86) Internationale Anmeldenummer: PCT/EP2012/051644
(87) Internationale Veröffentlichungsnummer: WO 2012/104332

(56) Entgegenhaltungen:
- EP-A1- 2 160 081
- WO-A1-2010/146438
- WO-A1-2011/110343
- DE-B1- 2 144 861
- FR-A1- 2 949 650
- US-A1- 2007 212 253
- US-A1- 2012 143 121
- JASON D HARPER ET AL: "Low-Temperature Plasma Probe for Ambient Desorption Ionization", ANALYTICAL CHEMISTRY, AMERICAN CHEMICAL SOCIETY, US, Bd. 80, Nr. 23, 4. November 2008 (2008-11-04), Seiten 9097-9104, XP008144746, ISSN: 0003-2700, DOI: 10.1021/AC801641A [gefunden am 2008-11-04]
- Zimmerman J.: "PLASMA GESUNDHEITSFÜRSORGE", MPE-Max-Planck-Institut für extraterrestrische Physik initiiertes Projekt , Januar 2011 (2011-01), XP002719226, Gefunden im Internet: URL:http://www.mpe.mpg.de/882068/PlasmaMed izin_Broschuere.pdf [gefunden am 2014-01-24]
- DATABASE GNPD [Online] MINTEL Januar 2000 'Sport Deodorant' Database accession no. 10064335

## Beschreibung

Die Erfindung umfasst ein Nicht-therapeutisches Verfahren zur Desodorierung, das sich aus der Kombination der Auftragung einer kosmetischen oder dermatologischen Zubereitung und von Plasma ergibt.

In der Physik ist ein Plasma (gr. πλάσµα plásma"Gebilde") ein Gas, das teilweise oder vollständig aus freien Ladungsträgern, wie z.B. Ionen oder Elektronen, besteht.

Plasma beruht auf einem einfachen physikalischen Prinzip. Durch Energiezufuhr ändern sich die Aggregatzustände: aus fest wird flüssig, aus flüssig gasförmig. Wird einem Gas nun weitere Energie zugeführt, so wird es ionisiert und geht in den Plasmazustand als erweiterten Aggregatzustand über.
Ein Plasma kann nur durch äußere Energiezufuhr erhalten werden. Bleibt die Energieeinkopplung aus, so erlischt das Plasma, das heißt die positiven und negativen Ladungsträger rekombinieren zu neutralen Atomen, Molekülen oder Radikalen.

Eine Klassifizierung der höchst unterschiedlichen Formen von Plasma kann aufgrund mehrerer Kriterien vorgenommen werden. In der Natur vorkommende Plasmen variieren in ihrer Dichte um mehr als 10 Größenordnungen. Extrem hohe Dichte besitzt das Plasma im Sonneninneren, extrem niedrige Dichte herrscht in interstellaren Gasnebeln. Entsprechend extrem sind die Unterschiede in den physikalischen Eigenschaften von Plasmen. Ein Schlüsselparameter zur Unterscheidung von Plasmen ist der Druck des Gases, in welchem sich die ionisierten Teilchen bewegen. Dieses Hintergrundgas wird auch als Neutralgas bezeichnet.

### Es kann unterschieden werden zwischen

- Niederdruckplasmen
- Normaldruckplasmen bzw. Atmosphärendruckplasmen
- Hochdruckplasmen

Niederdruckplasmen werden in verdünnten Gasen erzeugt, deren Druck signifikant niedriger liegt als der Atmosphärendruck. Bei Hochdruckplasmen ist der Druck des Gases signifikant höher als der Atmosphärendruck. Ein typisches Beispiel sind so genannte Hochdrucklampen.

Normaldruckplasmen werden ungefähr bei atmosphärischem Druck erzeugt.

In der vorliegenden Anmeldung ist der Begriff Plasma als Normaldruckplasma, Atmosphärendruckplasma bzw. non-thermal Plasma zu verstehen.

Der Einsatz von Plasma in der Medizin hat sich in den letzten Jahren infolge des zunehmenden Verständnisses für komplexe Plasmaphänomene und der Entwicklung vielfältiger neuer Plasmaquellen zu einem innovativen interdisziplinären Forschungsgebiet mit großem Potential entwickelt. Während man sich früher nur die thermischen Eigenschaften der Plasmen (> 80 °C) für medizinische Zwecke zunutze machte, ist der Fokus nun vorrangig auf Anwendungsmöglichkeiten nicht-thermischer Plasmen unter Atmosphärendruck gerichtet. Von besonderem Interesse sind kalte Plasmen im Bereich der Krankenhaushygiene und bei der Behandlung diverser Haut- und Infektionserkrankungen.

In der Broschüre des Max-Planck-Institutes für extraterrestrische Physik "Plasma Gesundheitsfürsorge" (www.mpe.mpg.de/theory/plasma-med) befasst sich eine spezielle Entwicklung mit tragbaren, freiverkäuflichen Plasmageräten für den häuslichen Gebrauch zu einem erschwinglichen Preis. Als Anwendungen werden kosmetische Anwendungen bis hin zur Hygiene genannt.

Das so erzeugte Plasma ist ein teilweise ionisiertes Gas und umfasst:
- neutrales Gas
- geladene Teilchen
- angeregte Atome/Moleküle
- reaktive Komponenten, insbesondere Ozon
- Licht
- elektrisches Feld
- Hitze

Bekannt ist, dass Plasma bzw. diese genannten Bestandteile bakterizide, fungizide und viruzide Eigenschaften haben.
Als Anwendungsgebiete von Plasmaapplikationen in der persönlichen Hygiene sind daher benannt:
- Behandlung von Fußpilz
- Zahnpflege und Mundhygiene
- häusliche Hygiene
- Verringerung von sich vermehrenden Bakterien im Schweiß

Die DE 19929802 A1 beschreibt die desodorierende Behandlung von Collagenfilmen oder - schwämmen mit Ozon. Die Ozonbehandlung führt zu einer Geruchsverbesserung der vornehmlich aus dem Meer stammenden Collagenprodukte.

EP 1747258 A2 beschreibt die Beschichtung eines Wischtuches oder Lappen mit einem wirkstoffhaltigen Material, das aus einem plasmabehandelten Gemisch aus zerstäubten beschichtungsbildenden Stoffen und den Wirkstoffen besteht.

In den WO 2007031250 A1 und WO 2008138504 A1 werden Plasmaquellen zur Wundbehandlung beschrieben. Die damit erzielbaren Plasmen weisen eine geringe Temperatur und geringe elektromagnetische Strahlung auf.
WO 2010094307 A1 beschreibt eine Plasmaquelle zur in-vivo Sterilisation der Hand.

Eine geringe Temperaturentwicklung ist für die hautverträgliche Anwendung am Menschen zwingend notwendig. Die erfindungsgemäß angewendeten Plasmaquellen sind demzufolge nur solche, die für eine Anwendung am Menschen geeignet sind, wie die sog. low temperature oder non-thermal plasmasource, wie sie im Stand der Technik offenbart werden.

WO 2010022871 A1 beschreibt die Kombination eines non-thermal Plasmagerätes mit Additiven, die eine verbesserte Wundheilung verursachen. Die Additive werden gewählt aus Salzen, organischen oder anorganischen Substanzen, Biomolekülen, Proteinen oder Enzymen.

Es gibt verschiedene Technologien zur Plasma-Produktion, so z.B. durch Radiofrequenz oder Mikrowellen-induzierte Entladungen. Diese Geräte nutzen ein Edelgas (Ar, He, Ne) als Träger, das mit weiteren Gasen ergänzt werden kann, um eine andere chemische Zusammensetzung zu erreichen. Die Anwendungsmöglichkeiten erstrecken sich auf regenerative, prophylaktische und therapeutische Medizin.

Erfindungsgemäße Plasmen sind vornehmlich sog. indirekte Plasmen. Indirekte Plasmen werden zwischen 2 Elektroden produziert und dann erst als Gasstrom zum Zielgebiet transportiert (Sladek RE, Stoffels E. Deactivation of Escherichia coli by the plasma needle. J Phys D: Applied Physics. 2005; 38: 1716-21). Die meisten Geräte dieser Art erzeugen dünne (Millimeter-Durchmesser) Plasma-Jets. Größere Flächen können durch das Zusammenfügen vieler solcher Jets bzw. durch multi-Elektrodensysteme gleichzeitig behandelt werden. Es können deutlich größere Areale behandelt werden als bei direkten Plasmen. Außerdem ist der Abstand zwischen Gerät und Haut in gewissem Maße variabel, da die Haut nicht als Plasmaelektrode benötigt wird, so dass die Anwendung erheblich erleichtert wird.

Eine weitere Ausführung der Plasmatechnologie nutzt den Venturi-Durchfluss-Effekt: Hierbei handelt es sich um ein Plasma-Jet-Gerät, das in der Luft oder mit geeigneten "Gasmischungen und Zusätzen" zum Plasma-Design betrieben werden kann. Der spezielle Venturi-Durchfluss-Effekt ermöglicht eine Plasmaproduktion bei Atmosphärendruck in einer lokalen Unterdruck-Umgebung. Dies macht die Plasmaproduktion einfacher und effizienter. Die Anwendungsmöglichkeiten liegen in der Zahnheilkunde, Kosmetik und Chirurgie.

Ein sog. HandPlaSter®, ein Niedertemperatur-Plasmagerät mit BCD-Technik (≤ 0,5 W/cm², 18 kVpp, 12,5 kHz), das zur potentiellen Anwendung für die Händedesinfektion entwickelt wurde, ermöglicht in vitro innerhalb weniger Sekunden (< 10 s) eine Bakterienreduktion von über 5 log-Stufen.

Eine weitere Plattform-Technologie zur Plasmaerzeugung ist die Oberflächen-Mikro-Entladung (SMD - Surface Micro Discharge), bei der atmosphärisches Plasma über große Flächen produziert werden kann mit einem Energiebedarf von weniger als 0,5 W/cm². Die Elektrode kann in jeder beliebigen Form hergestellt und auf verschiedene Größen skaliert werden. Das Prinzip beruht auf der Oberflächen-Mikro-Entladung, bei der das Plasma durch zahlreiche Mikroentladungen erzeugt wird. Die Anwendungsmöglichkeiten erstrecken sich auf alle Bereiche der professionellen und persönlichen Hygiene, Lebensmittelhygiene und Kosmetik. Die voraussichtlichen Kosten sind abhängig von der speziellen Anwendung. Die SMD-Technologie ermöglicht Plasmaquellen für Low-Budget-Anwendungen. Ein auf der SMD-Technologie basierendes Handgerät ist mit einer aufladbaren Batterie ausgestattet. Das Plasma wird beispielsweise durch eine zylindrische SMD-Elektrode produziert. Ein Ventilator hinter der Elektrode sorgt für den Plasmafluss.

Nachteil bei der Plasma-Erzeugung und deren Anwendung in der Kosmetik ist ein charakteristischer Eigengeruch bei der Entstehung der reaktiven Plasmateilchen (ionisiertes Gas, wie z.B. Ozon). Ebenso sind eine effiziente Handhabung und eine gezielte kosmetische, insbesondere desodorierende, Wirkung nicht gegeben.

Weiterer Nachteil der Plasma-Technologie bzw. deren Anwendung in der Kosmetik ist, dass die SMD-Technologie eine hohe Spannung erfordert, damit es zur Oberflächen-Mikro-Entladung kommen kann. Beim Einsatz von Handgeräten mit aufladbaren Batterien kann es teilweise nicht zu einem ausreichenden Spannungsaufbau und somit zu einer nicht ausreichenden Entladung kommen. Da in diesem Fall zu wenig reaktive Plasmateilchen freigesetzt werden, kann eine ausreichende antibakterielle und damit desodorierende Wirksamkeit nicht garantiert werden.

Für die kosmetische Anwendung als Deodorant ist die Plasmatechnologie daher alleine nicht ausreichend. Der Verbraucher versteht im Allgemeinen unter einem Deodorant ein Produkt, das vor der Entstehung von Schweißgeruch schützt. Dieser Schutz wird meist durch antimikrobielle Bestandteile, die die Keimbesiedlung in der Achsel minimieren und so die Verstoffwechselung des an sich geruchlosen Schweißes verhindern, erreicht. Weitere Zusätze, wie Antitranspirantien, verhindern zusätzlich die Entstehung von Schweiß. Der Zusatz von Parfum sorgt dafür, dass das Deodorant gut duftet, um den Frischeeindruck und das wirksame Gefühl zu unterstützen. Die Plasma-Technologie alleine ist nicht in der Lage, alle Parameter eines verbraucher-relevanten Deo-Produktes zu erfüllen - lediglich eine Minimierung der Keimzahl und damit eine Verminderung der Entstehung unangenehmen Schweißgeruchs kann mittels des Plasmas erzielt werden.

Wünschenswert ist es daher, eine Applikationsform der Plasmatechnologie bereit zu stellen, die
- für den Anwender handhabbar ist,
- effizient kosmetisch, insbesondere desodorierend wirksam ist,
- die Nachteile der Plasmatechnik, wie z.B. Eigengeruch, vermindert bzw. vermeidet,
- die eventuell vor der Behandlung bereits vorhandene Schlechtgerüche am Körper, in der Achsel und/oder in oder an Textilien neutralisiert oder entfernt
- trotz Betriebs ohne dauerhafte Stromversorgung eine ausreichende Deo-Leistung vermittelt
- durch Textilien hindurch applizierbar ist.

Wünschenswert ist es weiterhin, ein Deodorant zur Verfügung zu stellen, das eine antimikrobielle Wirkleistung aufweist, um eine für den Konsumenten ausreichende Wirkung zu zeigen, dabei aber weder als chemischer Rohstoff auf der Haut verbleibt und somit keine Reizungen hervorruft, noch zu Schwierigkeiten/Instabilitäten/Freisetzungsproblematiken bei der Einarbeitung in kosmetische Zubereitungen mit sich bringt,

Erfindungsgemäß ist ein Verfahren zur Desodorierung gemäß Anspruch 1, in dem eine kosmetische oder dermatologische Zubereitung auf die Haut aufgetragen wird und zeitgleich oder anschließend auf den sich bildenden Hautfilm Plasma aufgetragen wird.
Entsprechend kann eine Desodorierung auch anderer Gegenstände, wie Textilien, durchgeführt werden.
Durch die Kombination einer kosmetischen und/oder dermatologischen Zubereitung mit erzeugten reaktiven non-thermalen Plasmateilchen wird zum einen eine für den Verbraucher ausreichende antimikrobielle und zum anderen eine geruchlich weniger stark wahrnehmbare Wirksamkeit erreicht, da ein Teil der geruchsverursachenden Plasmateilchen im Hautfilm, der sich durch den Auftrag der kosmetischen Zubereitung bildet, gebunden wird. Die kosmetische und/oder dermatologische Zubereitung kann dabei optional weitere bekannte Wirkstoffe, wie Antitranspirant-und Deodorantmittel, enthalten.
Das non-thermale Plasma wird bevorzugt durch SMD-Technologie erzeugt. Die Vorrichtungen zur Plasmaerzeugung können entsprechend den aus der Broschüre des Max-Planck-Institutes oder im Stand der Technik beschriebenen sogenannten handheld-Vorrichtungen ausgebildet sein.
Die Erfindung entwickelt den hier vorgestellten Ansatz weiter und bietet dem Anwender eine Möglichkeit einer effizient kosmetischen, insbesondere desodorierenden Anwendung ohne die Nachteile der Plasmatechnik, wie z.B. Eigengeruch.
Der Film, der sich durch Applikation einer kosmetischen und/oder dermatologischen Zubereitung ausbildet, besteht aus dem Fachmann bekannten Applikationssystemen für den Bereich der kosmetischen Zubereitungen, insbesondere Deodorantien oder Antitranspirantien.
Die Zubereitungen umfassen demzufolge ein oder mehrere kosmetische Bestandteile, die in der Lage sind, auf der Haut einen Film auszubilden, wie beispielsweise Lösemittel, Öle und/oder Wachse. Der Hautfilm bleibt idealerweise für einen längeren Zeitraum auf der Haut existent. Die kosmetischen und/oder dermatologischen Zubereitungen, die dieser Erfindung zugrunde liegen, enthalten daher Öle, die aufgrund ihrer chemischen Eigenschaften ganz oder teilweise auf der Haut verbleiben und dort einen semi-okklusiven Film ausbilden. Die Filmbildung auf der Haut wird aufgrund weiterer Bestandteile wie z.B. Emulgatoren bevorzugt stabilisiert.
Durch Eindringen der ionisierten, reaktiven Teilchen des Plasmas in diesen semi-okklusiven Hautfilm kommt es zu einem Lösen der reaktiven Plasma-Bestandteile im Film sowie zu einer Kettenreaktion mit den Bestandteilen des Zubereitungsfilms. Durch die Reaktion des Plasmas mit Zubereitungsbestandteilen können zum einen weitere reaktive Spezies entstehen und zum anderen der technische Eigengeruch des Plasmas deutlich reduziert werden. Erfindungsgemäß umfasst das aufzutragende Plasma insbesondere die sich dadurch bildenden Bestandteile, wie neutrales Gas, geladene Teilchen, angeregte Atome/Moleküle, reaktive Komponenten.
Als reaktive Komponente bildet sich bei der Plasmaapplikation u.a. Ozon.
Eine vorteilhafte Ausführungsform ist, wenn Ozon als reaktiver Bestandteil auf eine auf die Haut aufgetragene kosmetische oder dermatologische Zubereitung aufgetragen wird und in Kombination dann den erfindungsgemäßen Hautfilm bildet. Der Vorteil der Minderung des Geruches des Ozons ist auch hier erfindungsgemäß in gleicher Weise beobachtbar.

Der desodorierende Hautfilm ist somit erhältlich aus der Kombination von auf der Haut aufgetragener kosmetischer und/oder dermatologischer Zubereitung und darauf aufgetragenem Plasma.

Der Hautfilm bildet sich erfindungsgemäß durch Applikation der Zubereitung und gleichzeitigem und/oder nachträglichem Auftragen des Plasmas aus.

Vorteilhafterweise werden der Zubereitung Parfum oder einzelne Parfumkomponenten zugesetzt, um so den Eigengeruch des Plasmas und/oder Ozons weiter vorteilhaft zu überdecken.

Antitranspirantien oder Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.
Im allgemeinen Sprachgebrauch erfolgt nicht immer ein klare Trennung der Begriffe "Deodorant" und "Antitranspirant". Vielmehr werden - insbesondere auch im deutschsprachigen Raum - Produkte zur Anwendung im Achselbereich pauschal als Desodorantien bzw. "Deos" bezeichnet. Dies geschieht unbeachtlich der Frage, ob auch eine antitranspirante Wirkung vorliegt. Antitranspirantien (AT) sind schweißverhütende Mittel, die - im Gegensatz zu den Desodorantien, die im Allgemeinen eine mikrobielle Zersetzung von bereits gebildetem Schweiß verhindern - die Absonderung von Schweiß überhaupt verhindern sollen.

Im Gegensatz zu den Antitranspirantien bewirken reine Desodorantien keine aktive Beeinflussung der Schweißsekretion, sondern lediglich die Steuerung bzw. Beeinflussung des Körper- bzw. Achselgeruchs (Geruchsverbesserungsmittel). Gängige Wirkmechanismen hierzu sind antibakterielle Effekte, Geruchsneutralisation (Maskierung), Beeinflussung von bakteriellen Metabolismen, die reine Parfümierung wie auch die Verwendung von Vorstufen bestimmter Parfümkomponenten, welche durch enzymatische Umsetzung zu wohlriechenden Stoffen verstoffwechselt werden.
Zubereitungen können auch Stoffe enthalten, die den mikrobiellen Abbau des Schweißes hemmen, wie z.B. Triclosan. Triclosan wirkt gegen gram-positive und gram-negative Keime sowie gegen Pilze und Hefen, woraus eine desodorierende, jedoch keine antitranspirante Wirkung resultiert, da aus der Beeinflussung der bakteriellen Hautflora keine Beeinflussung der Schweißsekretion abzuleiten ist.

In der erfindungsgemäßen Kombination wirken das Plasma bzw. seine Bestandteile, wie z.B. das Ozon, antibakteriell und vermindern so die geruchsintensive Zersetzung des Schweißes durch Mikroorganismen.
Tests haben jedoch gezeigt, dass allein mit einer Plasmabestrahlung ein für den Verbraucher wahrnehmbarer desodorierender langfristiger Effekt nicht immer erreicht werden kann.
Hier setzt die Erfindung an, indem die Plasmawirkung durch Auftragung einer kosmetischen Zubereitung den desodorierenden Effekt unterstützt. Erfindungsgemäß ist hierbei jedoch erst die Kombination, die sich im Hautfilm ausbildet und der das aufgenommene Plasma bzw. dessen Bestandteile umfasst. Der Hautfilm zeigt überraschende Vorteile und wirkt gleichzeitig synergistisch hinsichtlich der Deoleistung.
Durch Plasma erzeugte ionisierte Gase sind antimikrobiell wirksam. Diese Wirksamkeit kann für die Desodorierung des Körpers, insbesondere der Achsel ggf. nicht ausreichend sein. Der Film der kosmetischen und/oder dermatologischen Zubereitung wirkt aber auf die Deoleistung des Plasmas unterstützend, indem die antimikrobiellen Gase länger am Wirkort verbleiben können. Zusätzlich mindert oder verhindert die Zubereitung den unangenehmen Plasma-/Ozon-Geruch.

Die kosmetische oder dermatologische Zubereitung muss nicht zwingend Deodorantwirkstoffe umfassen. Allein die Kombination von Zubereitung mit Plasma und die Ausbildung des Hautfilms führen zu einer Steigerung des Deo-Effektes des Plasmas, eine Art Bündelung der Deo-Plasmawirkung am Applikationsort.

Wenn die antimikrobielle Wirksamkeit das Plasmas nicht ausreichend sein sollte aufgrund einer zu starken Keimbelastung auf der Haut bzw. in der Achsel, ist die vorherige/gleichzeitige Applikation einer bevorzugt antimikrobiellen kosmetischen und/oder dermatologischen Zubereitung von Vorteil, da sich im entstandenen Hautfilm weitere reaktive Plasmateilchen ausbilden und antimikrobiell wirken können. Antimikrobiell wirksam sind also entweder alleine die reaktiven Plasmateilchen oder die Kombination aus den ionisierten Gasen mit wirksamen Bestandteilen des Produktfilms. Die antimikrobielle Wirkung zeichnet sich durch eine geringere Keimbesiedlung in der Achsel aus, was per Definition zu verminderter Entstehung von Achselgeruch führt.

In einer bevorzugten Ausführungsform erfolgen die Applikation der Plasmabestrahlung und die Auftragung der Zubereitung zeitgleich.

Es ist allerdings ebenso möglich, dass die Plasmaapplikation erst nach dem Auftragen der Zubereitung auf die Haut erfolgt. Der zeitliche Abstand kann dabei frei variierbar gestaltet werden.
Ebenso ist eine Applikation durch die Kleidung möglich.

Dabei wird beispielsweise die kosmetische und/oder dermatologische Zubereitung mittels bekannter Applikationsformen in der Achsel aufgetragen. Da die Kombination mit dem Plasma oder seinen Bestandteilen, z.B. Ozon, auch nachträglich, also zeitlich verzögert, erfolgen kann, ist es dem Konsumenten möglich, dann zu einem späteren Zeitpunkt Plasma zu applizieren. Dadurch besteht ebenso die Möglichkeit, dass das Plasma, also die ionisierten Gase, durch die Kleidung angewendet wird. Vorteil dabei ist, dass der Konsument bereits anfänglich durch die kosmetische und/oder dermatologische Zubereitung vor unangenehmem Körpergeruch geschützt ist, diesen Schutz später bei Bedarf aber unkompliziert durch die Kleidung durch Plasmabestrahlung auffrischen, erneuern bzw. intensivieren kann. Ein weiterer Vorteil liegt darin, dass unangenehme Gerüche, die sich in den Textilien festgesetzt haben und einen Auffrischungs-Bedarf auslösen, durch die PlasmaBehandlung ebenfalls minimiert bzw. eliminiert werden können.

Auch eine gleichzeitige Applikation von Plasma und Zubereitung kann durch die Kleidung erfolgen. Dann handelt es sich bei der kosmetischen Zubereitung vorteilhaft um ein Aerosol, welches durch die Kleidung hindurch dringen kann.

Kosmetische oder dermatologische Zubereitungen zur topischen Applikation sind vielfältig bekannt. Bevorzugt werden Emulsionen, (wässrig-)alkoholische Lösungen, Gele,

Suspensionen in Form von Stiften oder Aerosolen, Seifen, Puder, Kristalle u.ä, verwendet. Als Applikationsformen sind Cremes, Roll-ons, Salben, Tonics, Stifte, Aerosole, Pumpsprays, Puder, Kristalle, Tücher u.ä. erfindungsgemäß möglich, insbesondere in Form von einer Creme, Lotion, Salbe, Gel, Emulsion oder (wässrig)-alkoholischer Lösung.

Bevorzugt werden die Zubereitungen in Form von Aerosolen, Stiften oder Emulsionen (Roll-ons) angewendet.

Die kosmetischen Zubereitungen umfassen ein oder mehrere okklusive Öle. Die Öle dienen neben der längerfristigen Ausbildung des Hautfilms auch zur besseren Bündelung des Plasmas bzw. seiner Bestandteile im sich bildenden Hautfilm. Umfasst die Zubereitung ein oder mehrere okklusive Öle, führt das zu einem verbesserten Hautfilm, der auch über einen längeren Zeitraum aufgrund der Okklusivität auf der Haut verbleiben kann und in dem die Deowirkung dann ggf. erfindungsgemäß durch Plasmabestrahlung aufgefrischt werden kann.

Bevorzugt sind Öle zu wählen aus der Gruppe der nicht flüchtigen Öle, bevorzugt aus der Gruppe der verzweigten und unverzweigten Kohlenwasserstoffe, der Silikonöle, der Dialkylether, der Dialkylcarbonate, der Gruppe der gesättigten oder ungesättigten, verzweigten Alkohole sowie der Fettsäuretriglyceride, namentlich der synthetischen oder natürlichen Triglycerinester gesättigter und/oder ungesättigter, verzweigter und/oder unverzweigter Alkancarbonsäuren einer Kettenlänge von 8 bis 24, der Gruppe der Ester aus gesättigten und/oder ungesättigten, verzweigten und/oder unverzweigten Alkanmono- oder Dicarbonsäuren einer Kettenlänge von 1 bis 44 C-Atomen und gesättigten und/oder ungesättigten, verzweigten und/oder unverzweigten Alkoholen oder Diolen einer Kettenlänge von 1 bis 44 C-Atomen, aus der Gruppe der Ester oder Diester aus aromatischen und/oder nicht aromatischen Carbonsäuren und gesättigten und/oder ungesättigten, verzweigten und/oder unverzweigten Alkoholen (einwertig oder mehrwertig) einer Kettenlänge von 1 bis 30 C-Atomen, sofern die Ölkomponente oder die Gesamtheit der Ölkomponenten bei Raumtemperatur eine Flüssigkeit darstellen.

Als okklusive Öle werden Öle gewählt aus der Gruppe: Dimethicon, Phenyltrimethicon, Sonnenblumenöl, Rapsöl, PPG-14 Butylether, C12-15 Alkylbenzoat, Capric/Caprylic Triglycerid, Isopropyl Myristate, Isopropylpalmitat, Isopropyl Stearate, Cetearyl Ethylhexanoate, Hydrogenated Polydecene, Paraffinum Liquidum, PPG-15 Stearylether, Avocadoöl (Persea Gratissima Oil), Glycine Soja (Soybean) Oil, Olivenöl, außerdem Guerbet-Alkohole wie beispielsweise Hexyldecanol, Octyldodecanol und 2-Ethylhexylalkohol, ferner auch Guerbetalkoholester, sowie Mischungen aus Guerbetalkoholen und Guerbetalkoholestem (wie z.B. Hexyldecanol und Hexyldecyllaurat).

Insbesondere vorteilhaft zu verwendende Öle sind Dimethicon, Phenyltrimethicon, Sonnenblumenöl, Rapsöl, PPG-14 Butylether, C12-15 Alkylbenzoat, Capric/Caprylic Triglycerid, Isopropylpalmitat, PPG-15 Stearylether.

Es ist davon auszugehen, dass Plasma bzw. Plasma-Bestandteile aufgrund ihrer antimikrobiellen Wirkleistung einen Deo-wirksamen Effekt hervorrufen können. Da dazu individuelle Einstellungen am Plasmaerzeuger notwendig wären und diese schwierig zu finden bzw. bereitzustellen sind, ist die erfindungsgemäße Kombination mit einer kosmetischen und/oder dermatologischen Zubereitung vorteilhaft bei der Verbesserung, Verlängerung, Intensivierung sowie Auffrischung der Deo-Wirksamkeit.

Da Plasma nur antimikrobiell wirksam ist, kann durch alleinige Anwendung von Plasma auch nur eine Deo-Wirksamkeit erzielt werden. Da der Verbraucher häufig unter einem Deodorant ein Produkt versteht, das auch gegen die Schweiß-Entstehung wirkt, also antitranspirant (AT) wirksam ist, ist es von Vorteil, wenn diese AT-Leistung durch die erfindungsgemäße Kombination mit der kosmetischen und/oder dermatologischen Zubereitung zusätzlich erbracht wird. Durch diese zusätzlichen Wirkstoffe in der kosmetischen Zubereitung bzw. durch die Kombination dieser Zubereitung und der Plasma-Applikation wird der vom Verbraucher erwartete Deo-Effekt nochmals verbessert, verlängert, aufgefrischt und/oder intensiviert.

Vorteilhaft umfasst die Zubereitung ein oder mehrere Deodorant-, Antitranspirantwirkstoffe und/oder Parfums, vorteilhaft sowohl AT- Wirkstoffe als auch Parfum.

In einem Sniff-Test konnte ein Nachteil bei der Plasmaapplikation festgestellt werden. Es konnte nachgewiesen werden, dass ein Plasma-produzierendes Gerät einen signifikant höheren technischen Geruch absondert als ein vergleichbares Placebo-Gerät (reiner Luftstrom ohne Erzeugung von Plasma). Dieser technische Plasma-Geruch wird erfindungsgemäß durch die Kombination mit einer kosmetischen und/oder dermatologischen Zubereitung absorbiert und damit wahrnehmbar reduziert bzw. ganz vermieden.

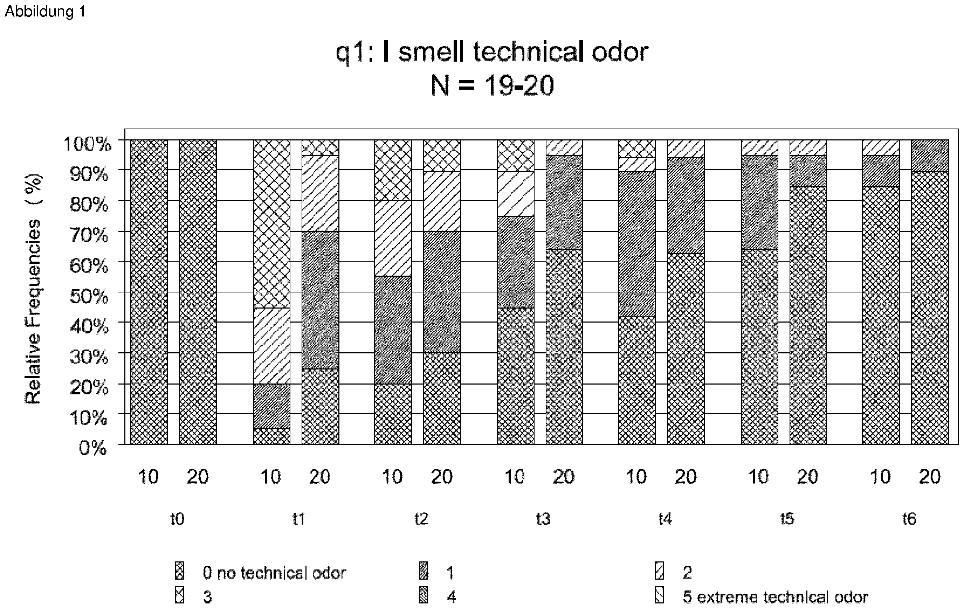

Abbildung 1 zeigt das Ergebnis des Sniff-Tests. Er zeigt, dass Probanden den technischen Geruch des Plasmas (10) gegen den des Placebos (20) signifikant differenzieren können.

Durch die Applikation einer kosmetischen und/oder dermatologischen Zubereitung wird bei gleichzeitiger oder nachträglicher Aufbringung von ionisierten Gasen eines mittels einer BCD-Elektrode produzierten Plasmas der Eigengeruch dieses durch Ausbildung weiterer geladener Teilchen, angeregter Atome/Moleküle und reaktiver Komponenten im durch Applikation einer kosmetischen und/oder dermatologischen Zubereitung entstandenen Hautfilm deutlich vermindert.

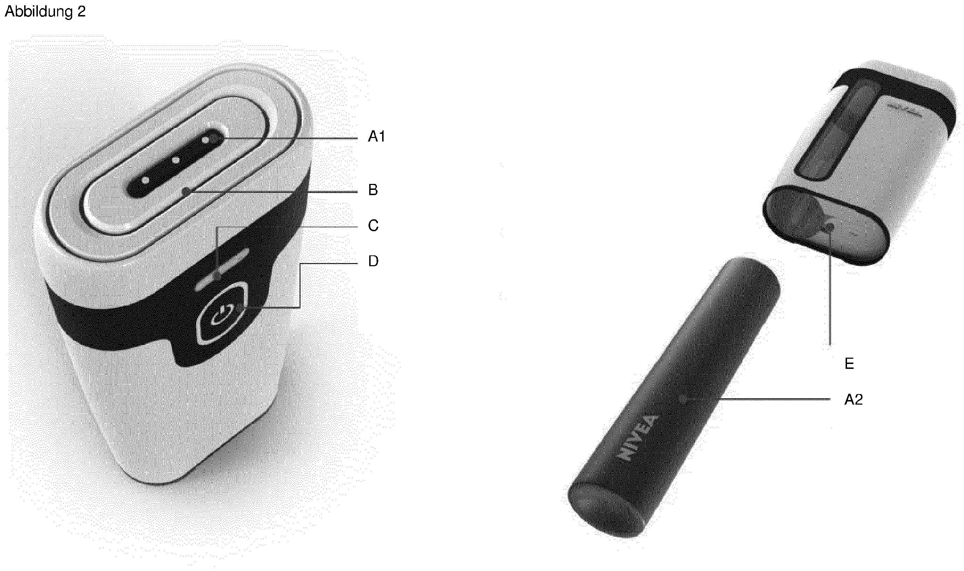

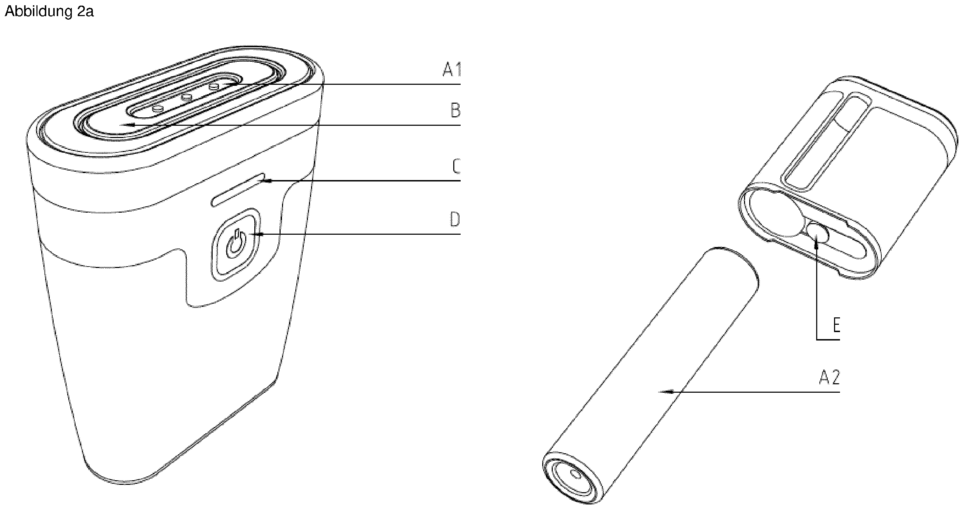

Vorteilhaft umfassen die kosmetischen oder dermatologischen Zubereitungen ein oder mehrere Parfüminhaltsstoffe, AT-Mittel wie z.B. AluminiumVerbindungen, vicinale Diole (wie in DE 10200904269 A1 beschrieben), bevorzugt 1,2-Propylenglykol, und/oder Salze von 1,1-Dimethyl-piperidinium oder 1,1-Dimethylpyrrolindium Verbindungen wie bspw. 4-[(2-Cyclopentyl-2-hydroxyphenylacetyl)oxy]-1,1-dimethyl-piperidiniumbromid oder andere Salze, 4-[(2-Cyclohexyl-2-hydroxyphenylacetyl)oxy]-1,1-dimethyl-piperidiniumbromid oder andere Salze, 4-[(3-Methyl-2-hydroxyphenylpentanoyl)oxy]-1,1-dimethyl-piperidiniumbromid oder andere Salze, 4-[(3-Methyl-2-phenylpentanoyl)oxy]-1,1-dimethyl-piperidiniumbromid oder andere Salze oder auch 3-[(2-Cyclopentyl-2-hydroxyphenylacetyl)oxy]-1,1-dimethylpyrrolidiniumbromid, pflegende Inhaltsstoffe wie beispielsweise Avocadoöl, Stoffe zur Erleichterung bzw. Reduzierung der Achselrasur wie z.B. Panthenol und Hamamelis-Extrakte, weitere Deowirkstoffe wie beispielsweise Octenidine dihydrochloride, Ethylhexylglycerin, Butyloctansäure, Polyaminopropyl Biguanide.
Vorteilhaft sind die den Hautfilm bildende Zubereitung und die Plasmaquelle in einer Vorrichtung enthalten.
Der sich bildende Hautfilm wird so durch eine Vorrichtung erbracht, in der die Zubereitung enthalten ist und die Plasma erzeugen kann.
Bei den Plasma-Geräten, die dieser Erfindung zugrunde liegen, handelt es sich vorteilhaft um kleine, gut handhabbare, mittels aufladbarer Batterien betriebene Geräte, die über BCD-Elektroden Plasma erzeugen. Zur Anwendung dieser Geräte muss der Verbraucher lediglich einen Starter auslösen, der zur Produktion des Plasmas führt.
Nachfolgend beschriebene Applikationssysteme veranschaulichen die erfindungsgemäße Kombination des erfindungsgemäßen Hautfilms.
Zeitgleich mit Auslösen des Starters des Plasmagerätes kann beispielsweise eine Aerosolapplikation initiiert werden (Abbildung 2).

Ein Applikator gemäß Abbildung 2 bzw. 2a umfasst innerhalb des Gehäuses eine Plasmaquelle sowie ein Aerosolbehältnis (A2) mit einer kosmetischen Zubereitung. A1 zeigt den Austritt der Aerosolsprühdüsen, B den Plasmaaustritt. Anwendungsvorteilhaft sind mit C eine Ladestandsanzeige der integrierten Batterie angegeben und mit D eine Mechanik, ein Ein/Aus-Schalter als auch eine Regulierung der Plasmaintensität und/oder des Aerosolsprühstoßes.
E ist eine Verriegelung für den Austausch des Zubereitungsbehältnisses (A2).

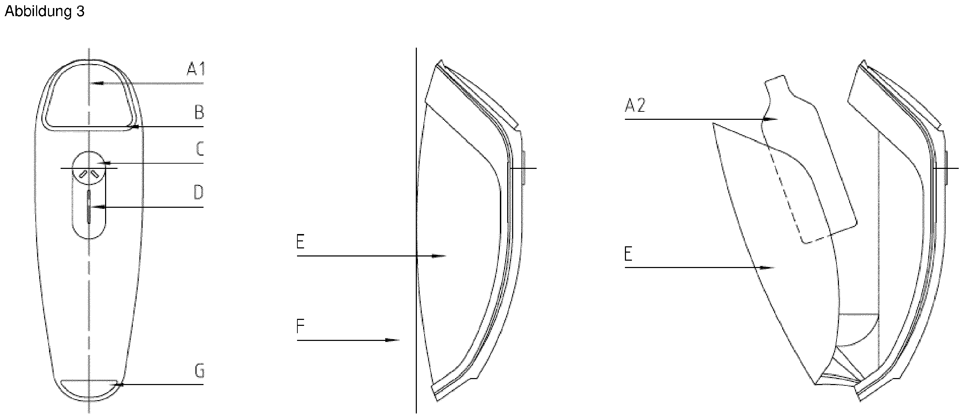

Abbildung 3 zeigt eine weitere Vorrichtung zur Applikation von Plasma und Aerosolzubereitung in der Aufsicht und Seitenansicht. A1 zeigt die Öffnung des Aerosolbehältnisses (A2), das austauschbar über eine Klappe (E) in der Vorrichtung integriert werden kann. B zeigt den Plasmaaustritt, C Mechanik bzw. Ein/Aus-Schalter, D Ladestandsanzeige, F Kontakte für Ladestation, G Lufteintritt.

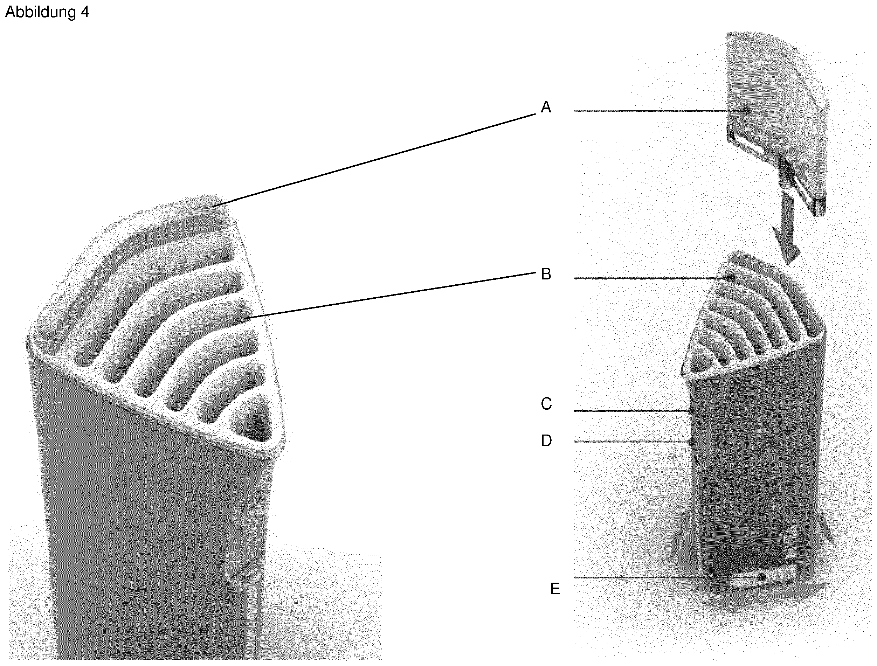

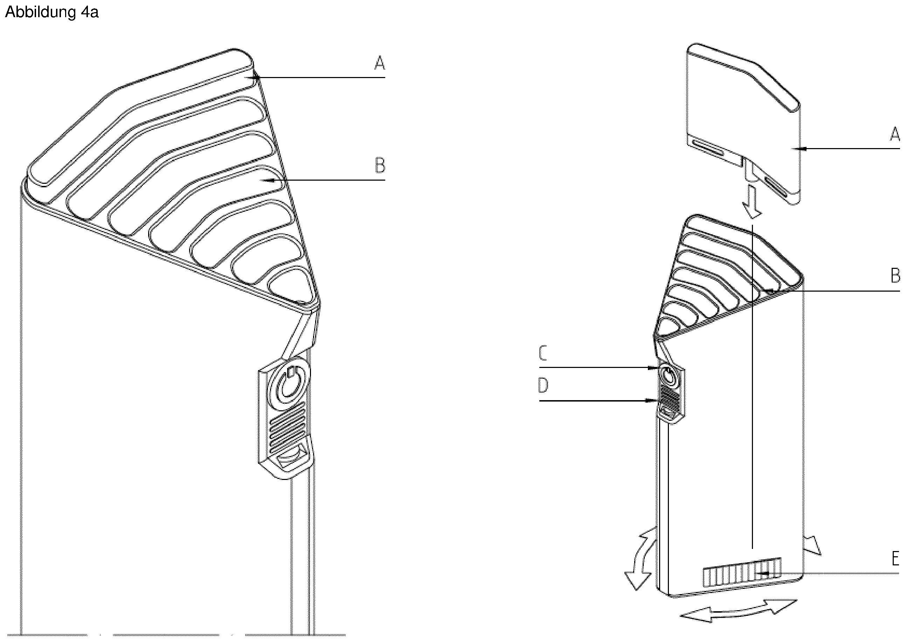

Abbildung 4 zeigt eine Vorrichtung mit kombinierter Applikation von Plasma und Stiftzubereitung. A zeigt den Stick, die Stiftzubereitung auf einem PE-Träger mit Gewindestange zum Ein- und Ausfahren aus der Vorrichtung. B ist der Plasmaaustritt, C Mechanik bzw. Ein/Aus-Schalter, D Ladestandsanzeige, F ist ein Raupenkettenband zur Höhenverstellung des Sticks.
Der Stick (A) wird, wie gewohnt, durch Reiben am Anwendungsort appliziert. Gleichzeitig kann über die neben stehende Applikationsfläche Plasma (B) ausgetragen werden.

Die Abbildungen 2a bzw. 4a zeigen die vorteilhaften Vorrichtungen der Abbildungen 2 bzw. 4 als Skizze.

Beide Produkte, Plasma und kosmetische und/oder dermatologische Zubereitung, können aber auch getrennt voneinander appliziert werden, wobei erfindungsgemäß zunächst die kosmetische und/oder dermatologische Zubereitung am Anwendungsort aufgetragen wird. Eine anschließende Zugabe des Plasmas erbringt den Deo-wirksamen Bestandteil bzw. verstärkt den bereits vorhandenen Deo-aktiven Teil der Zubereitung. Der "Deo-aktive" Teil der Gesamtkombination im Hautfilm besteht entweder aus den durch Plasma induzierten ionisierten Gasen alleine oder der Kombination aus den durch Plasma induzierten ionisierten Gasen (= antimikrobiell wirksam) und weiteren Deo-wirksamen Bestandteilen der kosmetischen und/oder dermatologischen Zubereitung (wie z.B. ein oder mehrere Parfümbestandteile, Deo-/AT-Mittel wie beispielsweise Aluminiumsalze und/oder weitere antimikrobielle Wirkstoffe).

Durch Applikation von kosmetischer Zubereitung und gleichzeitigem und/oder nachträglichem Applizieren von ionisierten Teilchen aus der Plasmaquelle kommt es während des Auftrags auf der Oberfläche/Haut in der Zubereitung zur Ausbildung weiterer geladener Moleküle und reaktiver Komponenten. Dies bewirkt eine Verstärkung der eigentlichen Plasma-Leistung. Damit ist die Kombination aus mit Plasma erzeugten ionisierten Gasen und einer kosmetischen und/oder dermatologischen Zubereitung wirksamer als ihre Einzelkomponenten.
Ein weiterer Vorteil der Anwendung von ionisierten Gasen über die Plasmatechnologie als antimikrobielles, kosmetisches Mittel ist die Anwendung durch Kleidungsstücke hindurch. Kosmetische Formeln sind nur direkt auf der Haut applizierbar, die ionisierten Gase aus der Plasmaquelle hingegen können auch Stoffe durchdringen und so auch zwischendurch notwendige Hygienemaßnahmen zulassen. D.h. die erfindungsgemäße Anwendung kann auch dadurch erfolgen, dass zunächst die kosmetische Zubereitung auf die Haut, in der Achsel, aufgetragen wird und danach, durchaus durch die Kleidung, dann die Plasmaapplikation erfolgt, Somit ist erfindungsgemäß ein "Auffrischen" der Deowirkleistung der kosmetischen Zubereitungen ohne aufwändiges zusätzliches Entkleiden, "Freimachen" der Achsel, Waschen etc. möglich.
Das erfindungsgemäße Verfahren, in dem eine kosmetische oder dermatologische Zubereitung auf die Haut und zeitgleich oder anschließend auf den sich bildenden Hautfilm Plasma aufgetragen wird, ist ein anwendungsfreundliches Desodorierungsverfahren.
Vorteilhaft ist darüber hinaus die Anwendung der Kombination zur Ausbildung eines desodorierenden Hautfilms, da die durch Plasma induzierten ionisierten Gase in der Lage sind, evtl. bereits entstandene Schlecht-/Schweißgerüche (auch auf/in der Kleidung) zu vermindern bzw. zu eliminieren und damit hat die Deo-Leistung durch Plasma nicht nur präventiven Charakter, sondern auch einen a posteriori.
Bestimmte Zusätze, wie z.B. Geruchsabsorber, die in Desodorantien bzw. Antitranspirantien zur Verminderung von unangenehmem Schweißgeruch eingesetzt werden, können hilfreich sein, den Eigengeruch der ionisierten Gase besser zu neutralisieren. Dies ist aber nicht zwingend notwendig, da die ionisierten Gase auch durch Abreaktion (Kettenreaktion) mit Bestandteilen der kosmetischen und/oder dermatologischen Zubereitung ihren charakteristischen Eigengeruch verlieren und somit erfindungsgemäß sind.

Als kosmetische oder dermatologische Zubereitungen können die im Stand der Technik bekannten Zubereitungen verwendet werden, vorteilhaft Deo- und/oder Antitranspirantzubereitungen in Form von Aerosol, Roll-ons oder Stiften. Erfindungsgemäß ist die Verwendung von auf der Haut aufgetragener kosmetischer oder dermatologischer Zubereitung und gleichzeitig oder nachfolgender Auftragung von nonthermalem Plasma zur Desodorierung gemäß Anspruch 4.

## Patentansprüche

1. Nicht-therapeutisches Verfahren zur Desodorierung indem eine kosmetische oder dermatologische Zubereitung auf die Haut aufgetragen wird und zeitgleich oder anschließend auf den sich bildenden Hautfilm non-thermales Plasma aufgetragen wird **dadurch gekennzeichnet, dass** die Zubereitung ein oder mehrere okklusive Öle umfasst und dass das oder die Öle gewählt werden aus der Gruppe Dimethicon, Phenyltrimethicon, Sonnenblumenöl, Rapsöl, PPG-14 Butylether, C12-15 Alkylbenzoat, Capric/Caprylic Triglycerid, Isopropyl Myristate, Isopropylpalmitat, Isopropyl Stearate, Cetearyl Ethylhexanoate, Hydrogenated Polydecene, Paraffinum Liquidum, PPG-15 Stearylether, Avocadoöl (Persea Gratissima Oil), Glycine Soja (Soybean) Oil, Olivenöl, Guerbet-Alkohole, Hexyldecanol, Octyldodecanol und 2-Ethylhexylalkohol, Guerbetalkoholester, Mischungen aus Guerbetalkoholen und Guerbetalkoholestem, Hexyldecanol und Hexyldecyllaurat.

2. Verfahren nach Anspruch 1 indem die Zubereitung in Form von einer Creme, Lotion, Salbe, Gel oder Emulsion aufgetragen wird.

3. Verfahren nach Anspruch 1 oder 2 indem die Zubereitung als Aerosol, Stift oder Roll-on aufgetragen wird.

4. Nicht-therapeutische Verwendung von auf der Haut aufgetragener kosmetischer oder dermatologischer Zubereitung und gleichzeitig oder nachfolgender Auftragung von nonthermalem Plasma zur Desodorierung **dadurch gekennzeichnet, dass** die Zubereitung ein oder mehrere okklusive Öle umfasst und dass das oder die Öle gewählt werden aus der Gruppe Dimethicon, Phenyltrimethicon, Sonnenblumenöl, Rapsöl, PPG-14 Butylether, C12-15 Alkylbenzoat, Capric/Caprylic Triglycerid, Isopropyl Myristate, Isopropylpalmitat, Isopropyl Stearate, Cetearyl Ethylhexanoate, Hydrogenated Polydecene, Paraffinum Liquidum, PPG-15 Stearylether, Avocadoöl (Persea Gratissima Oil), Glycine Soja (Soybean) Oil, Olivenöl, Guerbet-Alkohole, Hexyldecanol, Octyldodecanol und 2- Ethylhexylalkohol, Guerbetalkoholester, Mischungen aus Guerbetalkoholen und Guerbetalkoholestem, Hexyldecanol und Hexyldecyllaurat.

## Claims

1. Non-therapeutic method for deodorizing by applying a cosmetic or dermatological preparation to the skin and simultaneously or subsequently applying non-thermal plasma to the skin film that is forming, **characterized in that** the preparation comprises one or more occlusive oils and **in that** the oil(s) are selected from the group comprising dimethicone, phenyl trimethicone, sunflower oil, rapeseed oil, PPG-14 butyl ether, C12-15 alkyl benzoate, capric/caprylic triglyceride, isopropyl myristate, isopropyl palmitate, isopropyl stearate, cetearyl ethylhexanoate, hydrogenated polydecene, paraffinum liquidum, PPG-15 stearyl ether, avocado oil (Persea gratissima oil), glycine soya (soybean) oil, olive oil, Guerbet alcohols, hexyldecanol, octyldodecanol and 2-ethylhexyl alcohol, Guerbet alcohol esters, mixtures of Guerbet alcohols and Guerbet alcohol esters, hexyldecanol and hexyldecyl laurate.

2. Method according to Claim 1 in which the preparation is applied in the form of a cream, lotion, ointment, gel or emulsion.

3. Method according to Claim 1 or 2 in which the preparation is applied as an aerosol, stick or roll-on.

4. Non-therapeutic use of a cosmetic or dermatological preparation applied to the skin and simultaneous or subsequent application of non-thermal plasma for deodorizing, **characterized in that** the preparation comprises one or more occlusive oils and **in that** the oil(s) are selected from the group comprising dimethicone, phenyl trimethicone, sunflower oil, rapeseed oil, PPG-14 butyl ether, C12-15 alkyl benzoate, capric/caprylic triglyceride, isopropyl myristate, isopropyl palmitate, isopropyl stearate, cetearyl ethylhexanoate, hydrogenated polydecene, paraffinum liquidum, PPG-15 stearyl ether, avocado oil (Persea gratissima oil), glycine soya (soybean) oil, olive oil, Guerbet alcohols, hexyldecanol, octyldodecanol and 2-ethylhexyl alcohol, Guerbet alcohol esters, mixtures of Guerbet alcohols and Guerbet alcohol esters, hexyldecanol and hexyldecyl laurate.

## Revendications

1. Procédé non thérapeutique de désodorisation, selon lequel une préparation cosmétique ou dermatologique est appliquée sur la peau et un plasma non thermique est appliqué simultanément ou ultérieurement sur le film se formant sur la peau, **caractérisé en ce que** la préparation comprend une ou plusieurs huiles occlusives, et **en ce que** la ou les huiles sont choisies dans le groupe constitué par la diméthicone, la phényltriméthicone, l'huile de tournesol, l'huile de colza, l'éther butylique de PPG-14, le benzoate d'alkyle en C12-15, le triglycéride caprique/caprylique, le myristate d'isopropyle, le palmitate d'isopropyle, le stéarate d'isopropyle, l'éthylhexanoate de cétéaryle, le polydécène hydrogéné, la paraffine liquide, l'éther stéarylique de PPG-15, l'huile d'avocat (Persea Gratissima Oil), l'huile de soja (Glycine Soja Oil), l'huile d'olive, les alcools de Guerbet, l'hexyldécanol, l'octyldodécanol et l'alcool 2-éthylhexylique, les esters d'alcools de Guerbet, les mélanges d'alcools de Guerbet et d'esters d'alcools de Guerbet, l'hexyldécanol et le laurate d'hexyldécyle.

2. Procédé selon la revendication 1, selon lequel la préparation est appliquée sous la forme d'une crème, d'une lotion, d'un onguent, d'un gel ou d'une émulsion.

3. Procédé selon la revendication 1 ou 2, selon lequel la préparation est appliquée sous la forme d'un aérosol, d'un bâton ou par un flacon à bille rotative.

4. Utilisation non thérapeutique d'une préparation cosmétique ou dermatologique appliquée sur la peau et d'une application simultanée ou ultérieure d'un plasma non thermique pour la désodorisation, **caractérisée en ce que** la préparation comprend une ou plusieurs huiles occlusives, et **en ce que** la ou les huiles sont choisies dans le groupe constitué par la diméthicone, la phényltriméthicone, l'huile de tournesol, l'huile de colza, l'éther butylique de PPG-14, le benzoate d'alkyle en C12-15, le triglycéride caprique/caprylique, le myristate d'isopropyle, le palmitate d'isopropyle, le stéarate d'isopropyle, l'éthylhexanoate de cétéaryle, le polydécène hydrogéné, la paraffine liquide, l'éther stéarylique de PPG-15, l'huile d'avocat (Persea Gratissima Oil), l'huile de soja (Glycine Soja Oil), l'huile d'olive, les alcools de Guerbet, l'hexyldécanol, l'octyldodécanol et l'alcool 2-éthylhexylique, les esters d'alcools de Guerbet, les mélanges d'alcools de Guerbet et d'esters d'alcools de Guerbet, l'hexyldécanol et le laurate d'hexyldécyle.
